# EUROPEAN PATENT SPECIFICATION

(11) **EP 3 253 456 B1**
(45) Date of publication and mention of the grant of the patent: **12.05.2021**
(21) Application number: 16701828.2
(22) Date of filing: 29.01.2016
(51) Int. Cl.: A61Q 19/00

(54) **METHODS FOR THE PRODUCTION OF BIOPOLYMERS WITH SPECIFIC MOLECULAR WEIGHT DISTRIBUTION**
VERFAHREN ZUR HERSTELLUNG VON BIOPOLYMEREN MIT SPEZIFISCHER MOLEKULARGEWICHTSVERTEILUNG
PROCÉDÉS DE PRODUCTION DE BIOPOLYMÈRES À DISTRIBUTION DE POIDS MOLÉCULAIRE SPÉCIFIQUE

(30) Priority: 06.02.2015 EP 15154173
(43) Date of publication of application: 13.12.2017
(73) Proprietor: MedSkin Solutions Dr. Suwelack AG, 48727 Billerbeck (DE)
(72) Inventor: KUNZ, Michael, 48153 Münster (DE); KUHLMANN, Fabian, 48720 Rosendahl (DE); WIESWEG, Karin, 45739 Oer-Erkenschwick (DE); ELSINGHORST, Claudia, 48727 Billerbeck (DE)
(74) Representative: CH Kilger Anwaltspartnerschaft mbB
(86) International application number: PCT/EP2016/051955
(87) International publication number: WO 2016/124501

(56) References cited:
- EP-A1- 1 992 645
- EP-B1- 0 138 572
- ALEXANDRA SLOVIKOVÁ ET AL: "Preparation and modification of collagen-based porous scaffold for tissue engineering", CHEMICAL PAPERS, vol. 62, no. 4, 1 July 2008 (2008-07-01), pages 417-422, XP055130903, ISSN: 0366-6352, DOI: 10.2478/s11696-008-0045-8
- TOKITA Y ET AL: "Degradation of hyaluronic acid during freeze drying", POLYMER DEGRADATION AND STABILITY, BARKING, GB, vol. 55, no. 2, 1 January 1997 (1997-01-01), pages 159-164, XP027298643, ISSN: 0141-3910 [retrieved on 1997-01-01]

## Description

### FIELD OF THE INVENTION

The present invention is in the field of dermatology, pharmaceutics and cosmetics. In particular the invention is in the field of the production of pharmaceutically, dermatologically or cosmetically applicable substances and in the use and application thereof.

### BACKGROUND

Biopolymers, such as collagen, polysaccharides or hyaluronic acid are commonly used in cosmetic or dermatological compositions. In many cases these biopolymers are used as moisturizers or antioxidants. Common forms of administration are as cream, serum, patches, masks, balms, liquids or as an ointment.

Hyaluronic acid or hyaluronan for example is a biopolymer, which is widely distributed among the human tissue. It is an anionic, non-sulfated glycosaminoglycan comprising the following structure:

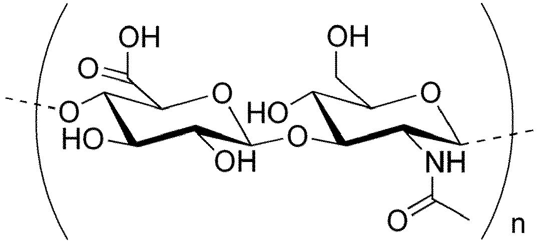

Hyaluronic acid has several medical uses, in particular in dermatology, and is commonly used in cosmetic products, in particular so called anti-ageing products.

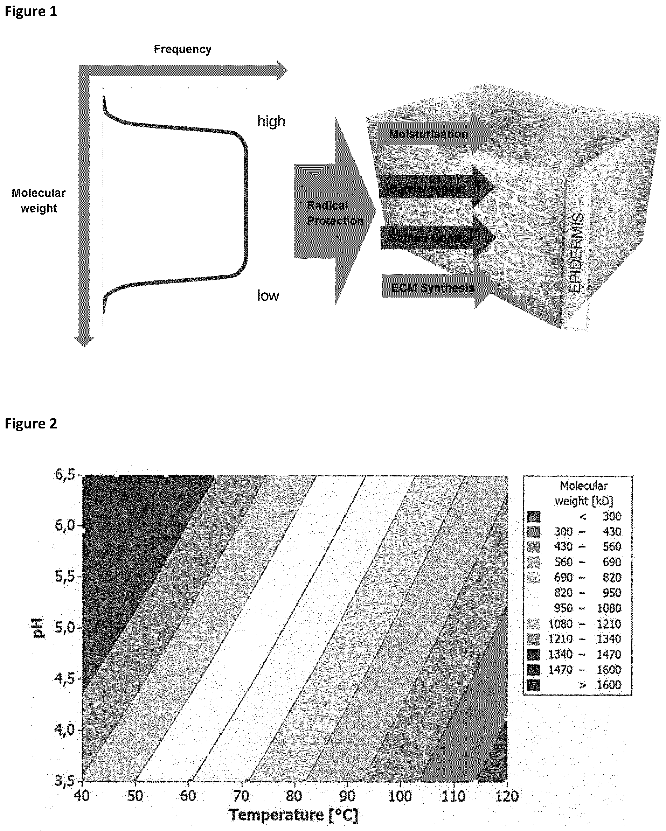

In general the bioactivity of biopolymers, such as hyaluronic acid is directly dependent on the average molecular weight of said biopolymers. Taking hyaluronic acid and its use in dermatology for example, the average molecular weight determines the depth of skin penetration and the potential dermatological effects of hyaluronic acid (see Figure 1).

It is known, that the biological functionality of biopolymers is dependent on their average molecular weight, several methods have been developed to generate biopolymers with defined average molecular weight.

EP 2 479 194 A2 describes the hydrolysis of hyaluronic acid on activated charcoal. EP 2 463 309 B1 and EP 1992 645 A1 describe several methods for the acidic hydrolysation of hyaluronic acid. Other methods involve the use of enzymatic hydrolysis and filtration (EP 0 138 572 B1) or the use of high temperatures and strong shearing forces (EP 1 987 153 B1).

The problem with all these methods is, that in particular hyaluronic acid needs extensive purification steps to remove the low molecular weight hyaluronic acids, which can be pro-inflammatory.

It is therefore necessary to provide a method for the efficient production of pure biopolymers with defined molecular weight distribution, in particular hyaluronic acid, which allows the control of the average molecular weight of the biopolymer and does not need any further additional purification steps.

### BRIEF DESCRIPTION OF THE INVENTION

The present invention relates to a method for the production of a biopolymer composition comprising at least one biopolymer, wherein the at least one biopolymer has a defined average molecular weight, the method comprising
(i) providing a first frozen composition comprising a biopolymer with a defined pH-value;
(ii) providing a second frozen composition comprising a biopolymer with a defined pH-value
(iii) combining the compositions in a reaction vessel without substantially mixing these with each other;
(iv) optionally freezing the compositions;
(v) lyophylizing the compositions comprising the biopolymers,
(v) optionally purifying and/or isolating the biopolymers;
wherein the maximum temperature during the lyophilization process is selected to facilitate a controlled and defined degradation of said biopolymer and wherein the pH-values of the first and second composition differ by at least 0.1 and/or wherein the first and second composition comprise different biopolymers.

The preferred biopolymers are biopolymers with high molecular weight.

More preferably the biopolymers are biopolymers with native high molecular weight.

The invention relates to a method for the production of a biopolymer composition comprising at least one biopolymer, wherein the at least one biopolymer has a defined average molecular weight, the method comprising
(i) providing a first composition comprising a biopolymer with high molecular weight with a defined pH-value between 1.5 and 8.5;
(ii) providing a second composition comprising a biopolymer with high molecular weight with a defined pH-value between 1.5 and 8.5
(iii) combining the compositions in a reaction vessel without substantially mixing these with each other;
(iv) optionally freezing the compositions;
(v) lyophylizing the compositions comprising the biopolymers,
(vi) optionally purifying and/or isolating the biopolymers;
wherein the temperature during the lyophilization process is selected to facilitate a controlled and defined degradation of said biopolymer and wherein the pH-values of the first and second composition differ by at least 0.1 or wherein the first and second composition comprise different biopolymers.

The invention further relates to the use of said method for the production of biopolymer compositions and to biopolymer compositions, which are produced by said method.

### DEFINITIONS

In the context of the present invention, biopolymers are polymers produced by living organisms.

Especially native high molecular weight biopolymers, which are preferably not technically or chemically modified, besides the common and native modifications, which occur in the living organism. As polymers, they are characterized by repetitive monomeric motives.

In general biopolymers are divided into three main classes: polynucleotides, polypeptides and polysaccharides. Within the context of this invention the term "biopolymer" only refers to polypeptides and polysaccharides. In the present invention the term "biopolymer" encompasses all naturally occurring modifications of biopolymers, e.g. glycosylation, partial hydrolysis or the attachment of lipids to polypeptides.

Polymers consisting of biological units, but not produced in a living organism, such as polylactic acid, are not considered biopolymers within the meaning of the invention. Biopolymers according to the above mentioned definition processed according to the present invention, are biopolymers in the context of the present invention.

Non-limiting examples for biopolymers according to the present invention comprise: collagens, starch, cellulose derivatives, glucosamino glycans, polysaccarides or fucoidanes.

In the context of the present invention a frozen composition refers to a composition, which is in a solid state of matter, regardless of its state of matter at 25 °C. In most embodiments a frozen composition is a composition in a liquid state of matter at 25 °C, which has been cooled down to a solid state of matter. In one embodiment cooling is done by shock-freezing in liquid nitrogen. Alternative ways of cooling are cooling and freezing in a freezer. In a preferred emboidment freezing is done at a temperature between -50 °C and -4 °C.

In the context of the present invention lyophilization or lyophilizing refers to a dehydration process, wherein water is removed by sublimation. Lyophilization is commonly referred to as freeze drying. In general lyophilization comprises three stages:
(i) freezing the composition to be dehydrated, wherein it is important that the composition is cooled below its triple point. The suitable freezing method is dependent on the components of the composition.
(ii) a primary drying phase, in which most of the water is removed, wherein the pressure is lowered to a few µbar or even lower. In this stage the temperature is usually adjusted to get the water sublimated. Preferably, but not necessarily, at this stage the temperature remains under 0°C.
(iii) a secondary drying phase, wherein the pressure is optionally reduced, down to the range of µbars and the temperature preferably raised above 0 °C to remove more strongly bound water.

In one embodiment of the invention, the temperature is controlled during the second drying phase. In another embodiment the temperature is controlled during the primary drying phase. In a particular embodiment the composition is dried using only one drying step, wherein the conditions correspond to the conditions of the second drying step. In an alternative embodiment the composition is dried using only one drying step, wherein the conditions correspond to the conditions of the first drying step.

Within the meaning of the present invention the "temperature during the sublimation process" refers to the temperature of the storage plate on which the composition is placed.

In the context of the present invention the term aqueous solution refers to a solution, wherein the solvent is water. Within the context of the present invention the term further refers to coarse or colloidal suspensions of components, for example non-water-soluble biopolymers or non-soluble cosmetic additions in water.

In the context of the present invention the term emulsion refers to mixtures of normally immiscible liquids. In the context of the present invention the term emulsion in particular refers to water-in-oil or oil-in-water emulsion. Preferably in the context of the present invention the emulsion is stabilized by the use of an emulsifying agent or emulsifier. Non-limited examples for emulsifying agents are lecithin, sodium stearoyl lactylate, polymers with emulsifying functionalities or detergents.

In the context of the present invention a reaction vessel is any suitable vessel for containing and processing the compositions. Preferably said vessel is suitable for freezing and lyophilization processes.

### DETAILED DESCRIPTION OF THE INVENTION

The inventors found surprisingly, that biopolymers can be subjected to controlled degradation during lyophilization processes, resulting in biopolymers with defined average molecular weight. In addition the inventors found, that the molecular weight distribution of the degraded biopolymer can be influenced by combining frozen compositions during the lyophilization process.

A first aspect of the present invention relates to a method for the production of a biopolymer composition comprising at least one biopolymer, wherein the at least one biopolymer has a defined average molecular weight, the method comprising
(i) providing a first frozen composition comprising a biopolymer with a defined pH-value;
(ii) providing a second frozen composition comprising a biopolymer with a defined pH-value
(iii) combining the compositions in a reaction vessel without substantially mixing these with each the compositions;
(iv) optionally freezing the compositions;
(v) lyophylizing the compositions comprising the biopolymers,
(vi) optionally purifying and/or isolating the biopolymers;
wherein the maximum temperature during the lyophilization process is selected to facilitate a controlled and defined degradation of said biopolymer and wherein the pH-values of the first and second composition differ by at least 0.1 and/or wherein the first and second composition comprise different biopolymers.

Combining the compositions without substantially mixing the compostions refers to a process, wherein the compositions are combined in one reaction vessel but retain individual concentrations of compounds and other properties, such as pH value. This could be done by providing and combining two frozen compositions or by combining two compositions with high viscosity by overlaying the compositions and freezing them prior to lyophilization.

In a preferred embodiment the compositions are frozen and then combined in one reaction vessel.

In a preferred embodiment the biopolymers are biopolymers with high molecular weight. In a more preferred embodiment the biopolymers are biopolymers with native high molecular weight.

The compositions comprising a biopolymer can be any kind of composition, provided said compositions comprise at least small amounts of water in addition to said biopolymer. Said composition may comprise additional biopolymers, i.e. mixtures.

The first and second compositions might comprise different biopolymers. In a preferred embodiment both compositions comprise the same biopolymer. In another preferred embodiment the first and second composition comprise only one biopolymer each.

In a preferred embodiment the biopolymers are biopolymers with high molecular weight. In a more preferred embodiment the biopolymers are biopolymers with native high molecular weight.

The method is in particular suitable for biopolymers selected from the group comprising hyaluronic acid, collagen, glucosamino glycans, polysaccharides and fucoidanes. In a preferred embodiment the biopolymer is a glucosamino glycan or polysaccarid. In a more preferred embodiment the biopolymer is selected from the group consisting of alginates, rhizobian gum, sodium carboxy methyl cellulose, pullulan, Biosaccharide Gum-1, glucomannane, beta-glucane, pectine, tamarindus indica seed polysaccharide and hyaluronic acid. In an even more preferred embodiment the biopolymer is sodium alginate or hyaluronic acid. In the most preferred embodiment the biopolymer is hyaluronic acid.

According to the invention, both compositions comprising a biopolymer are frozen aqueous solutions or emulsions.

The inventors had found in particular, that controlled conditions during the sublimation process and a control of the parameters of the compositions, e.g. salt contents, pH-value, vacuum, used emulsifying agents, allow the control of the average molecular weight of the degraded biopolymer. In particular the inventors found that combining compositions comprising the same biopolymer with different pH-values leads to a different distribution of the average molecular weight of the biopolymer, which allows differently defined weight distributions depending on the pH-values and/or volume of the compositions.

In one embodiment of the invention the first and/or second frozen composition comprising a biopolymer has a pH-value selected from a range between 1.5 and 8.5. In a preferred embodiment the pH value is selected from a range between 2.5 and 6.

If the first and second composition comprise the same biopolymer it is preferred that the pH value of the composition differs by at least 0.1. In a preferred embodiment the pH-value of first and second composition differs by at least 0.5. In a more preferred embodiment the pH-value of first and second composition differs by at least 1. In the most preferred embodiment the pH value of the first and second frozen composition differs by at least 2.

The inventors had found a direct correlation between the pH-value, the temperature during the sublimation process and the average molecular weight of the biopolymer. Figure 2 shows the correlation found for an analyzed hyaluronic acid.

It is evident that the average molecular weight of the final product of the processed biopolymer is directly dependent on the combination of temperature and pH value selected. The inventors further found that it is possible to stack or mix multiple frozen compositions to vary the molecular weight distribution of the final product.

In one embodiment of the invention the maximum temperature during the sublimation process is selected from the range of -40 °C to 150 °C. In a preferred embodiment the temperature is selected from the rage of 0 to 140 °C. In a more preferred embodiment the temperature is selected from the range of 60 to 130 °C. In the most preferred embodiment the temperature is 120 °C.

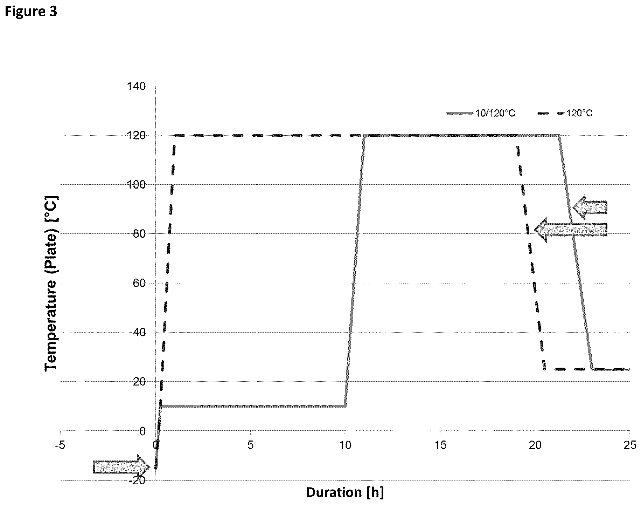

In an alternative embodiment the temperature during the sublimation process is varied during the lyphilization process. In a preferred first embodiment the sublimation is carried out at two temperatures. A schematic overview of preferred temperatures profile is shown in figure 3.

In one embodiment of the invention the sublimation process is carried out at two different temperatures. Preferably the first temperature is selected from the range of -30 ° to + 40 °C and the second temperature is selected from the range of 60 to 130 °C. In a preferred embodiment the first temperature is selected from the range of -20 to 20 °C and the second temperature is selected from the range of 80 to 120 °C. In a most preferred embodiment the first temperature is 10 °C and the second temperature is 120 °C.

In an alternative embodiment the temperature profile comprises more than two different temperatures. In an alternative embodiment the temperature profile comprises a continuous temperature gradient.

In one embodiment of the invention the pressure during the sublimation step is between 50 µbar and 800 µbar. In a preferred embodiment of the invention the pressure is between 75 µbar and 600 µbar, more preferably between 100 µbar and 400 µbar, even more preferably between 150 µbar and 300 µbar. In a most preferred embodiment the pressure during the sublimation step is 300 µbar.

The biopolymer compositions produced with the process might be purified or isolated from the composition, however it is preferred that no further purification or isolation step is performed. In the most preferred embodiment the biopolymer is directly suitable for further processing and/or use.

The present invention does not only relate to a method for the production of biopolymer compositions with defined average molecular weight, but also to the use of said method for the production of biopolymer compositions with defined average molecular weight and to the biopolymer compositions with defined average molecular weight produced with said method.

In a preferred embodiment the method is used for the productions of biopolymers with defined average molecular weight, which are selected from the group comprising hyaluronic acid, collagen, glucosamino glycans, polysaccharides and fucoidanes. In a more preferred embodiment the biopolymer is a glucosamino glycan. In a more preferred embodiment the method is used for the productions of biopolymers with defined average molecular weight selected from alginates, rhizobian gum, sodium carboxy methyl cellulose, pullulan, Biosaccharide Gum-1, glucomannane, beta-glucane, pectine, tamarindus indica seed polysaccharide and hyaluronic acid. In an even more preferred embodiment the the method is used for the productions of biopolymers with defined average molecular weight selected from sodium alginate or hyaluronic acid. In the most preferred embodiment the method is used for the production hyaluronic acid with a defined average molecular weight.

The inventors found that the present invention is suitable for the production of complex compositions, comprising biopolymers. These compositions comprise at least one biopolymer with defined average molecular weight and other optional components, such as dermatological, pharmaceutical or cosmetic ingredients and just need to be emulsified or dissolved to be used.

The complex compositions may comprise additional biopolymers or other polymers. Any composition is suitable, as long as the composition comprises additionally water.

In a preferred embodiment the first and/or second composition is an emulsion comprising:
(i) a biopolymer,
(ii) water,
(iii) optionally a pharmaceutically, dermatologically and/or cosmetically acceptable compounds and/or oils;
(iv) optionally an emulsifying agent
(v) optionally emollients.

In alternative embodiments the first and/or second frozen composition is a gel or a liquid with low to high viscosity.

The first and/ or second composition preferably contains further additional cosmetic, dermatological or pharmaceutical ingredients or additions. Non-limiting examples for these ingredients are emollients, cosmetically acceptable ingredients and dyes, perfumes or pharmaceutically active substances like panthenol.

In a preferred embodiment the first and second frozen composition are comprise the same compounds with the exception of the biopolymer or the pH-value. In the most preferred embodiment the first and second frozen composition comprise the same compounds and the same biopolymer and differ only in pH-value.

Non limiting examples for said ingredients or additions are: skin conditioning agents, skin-smoothing agents, agents for skin hydration, e.g. panthenol or panthothenol, natural moisturising factors, such as glycerine, lactid acid or urea. Alternatively a physical or chemical sunscreens, keratolitics, such as α- or β-hydroxy acids, α- or β-ketoacids. Further possible ingredients include radical catchers, anti-ageing agents, vitamins or derivatives thereof, e.g. vitamin C (ascorbic acid) or esters or glycosides thereof, antioxidants, such as catechins or flavonoids.

Further potential ingredients comprise resveratol, gluthation, ferulic acid, Q10, polyphenols, ceramides, saturated and or unsaturated fatty acids and there glycerides. Furthermore esters, such as wax esters, such as jojoba oil, triglycerides in general (neutral oil, argan oil, shea butter) or unsaponifiable components from plant oils.

Further ingredients comprise polysaccharides of vegetable, biotechnological or marine origin, as well as their hydrolysates. Other ingredients might include enzymes, e.g. bromelain, coenzymes, enzyme inhibitors, amino acids, natural and synthetic oligopeptides, peptides such as collagen and elastin, as well as their hydrolysates, neuropeptides, growth factors, alcaloids. In some embodiments the ingredients optionally include phytopharmaca such as aescin, ginsenosides, ruscogenine or aloin. Further polymers are alginates, cellulose derivatives, starch, chitosan, chondroitin sulfate, further synthetic biopolymers with biological function or compatibility

Non-limiting examples of cosmetic additions comprise skin lightening agents, inorganic or synthetic fillers or decorative substances, such as coloring pigments or dyes or particles. Some embodiments of the invention comprise substance for the cosmetic beautification of eyes, lips or face.

In some embodiments the first and/or second frozen composition further comprises therapeutically active agents, such as anti-acne or anti-rosacea agents, antimicrobial agents, such as silver and it's derivatives, iodine or PVP-iodine, antiperspirants, pain relieving substances such as lidocain or ibuprofen, adstringent substances, deodorizing compounds, antiseborrhoeic substances or antiseptics. Furthermore cells or cell components, such as autologous cells, allogenic cells, stem cells or platelet-rich plasma (PRP).

The first and/or second composition preferably contains other ingredients, e.g. stabilizers, preserving agents, to control the final parameters of the product, such a solubility or emulsifiability, mechanical stability, product viscosity or haptics.

In a particular embodiment of the present invention the first and second composition are combined in an appropriate container, which is suitable for the freezing and lyophilization process, as well as optionally able to serve as packaging for the lyophilized composition comprising at least one biopolymer with defined average molecular weight and defined weight distribution.

The invention further relates to the use of said method for the production of compositions comprising a biopolymer with defined average molecular weight and to compositions comprising biopolymers produced according to a method of the present invention.

In one embodiment of the present invention the final composition can serve as a basis for aqueous liquids, emulsions with low viscosity, serum-like liquids, masks, creams, cream masks, patches or segments for topical applications.

### FIGURE LEGENDS

**Figure 1****:** Correlation of average molecular weight, skin penetration and biological activity of hyaluronic acid.
**Figure 2****:** Correlation of pH, temperature and obtained average molecular weight obtained, when processing a composition comprising hyaluronic acid according to the present invention.
**Figure 3****:** Overview of suggested temperature profiles over time during the lyophilization process.
**Figure 4****:** Calibration curve to determine average molecular weight of hyaluronic acid, processed according to the present invention.
**Figure 5****:** Elution and molecular mass profiles of neutral oil (a) and Sepinov EMT-10 (b) processed according to the present invention.
**Figure 6****:** comparison of the elution profiles (A) and molecular mass profiles (B) of native hyaluronic acid and hyaluronic acid, lyophilized 120 °C.
**Figure 7****:** Comparison of the elution profile (A)and molecular mass profiles (B) of a mixture of hyaluronic acid, Sepinov EMT-10 and neutral oil, lyophilized at different temperatures.
**Figure 8****:** Molecular weight of lyophilized samples of 1 wt-% high molecular weight HA samples with pH adjusted in the range of 6.21 to 2.9.
**Figure 9****:** Molecular weight distributions of lyophilized samples of 1 wt-% high molecular weight HA samples with pH adjusted to 6.21 and 2.9.
**Figure 10****:** Molecular weight of hyaluronic acid containing emulsions lyophilized at different process temperatures.
**Figure 11****:** Molecular weight distributions of lyophilized samples of 1 wt-% high molecular weight HA samples with pH adjusted to 6.21 to 2.9 stacked at different volume ratios
**Figure 12****:** Molecular weight distributions of lyophilized samples of 1 wt-% pullulan samples with pH adjusted to 4.9 to 3.5 stacked at different volume ratios
**Figure 13**: Molecular weight distributions of lyophilized samples of 1 wt-% sodium alginate samples with pH adjusted to 3.5 and 7.15 stacked at different volume ratios

### EXAMPLES

### Example 1: Controlled degradation of hyaluronic acid

Deionized water is transferred to 1 I lab reactor and stirred at 75 °C. Hyaluronic acid powder is added and stirred at 75 °C at 700 rpm for 15 min until the material is dissolved. The emulsifier component is added and stirred at 50°C for 15 min at 1400 rpm under reduced pressure (200 µbar). The oil component is added and stirred at 1400 rpm/45 °C/200 µbar for 10 min and subsequently for 5 min at 2100 rpm/45 °C/200 µbar. The received emulsion is cooled to room temperature and transferred to 10 ml glass vials and stored overnight at ambient conditions. Samples were frozen in a deep freezer for minimum 16 h and subsequently lyophilized up to maximum target temperature.

As a proof of principle hyaluronic acid was processed according to the invented method. Herein, pure hyaluronic acid, and compositions of hyaluronic acid with MCT neutral oil and Sepinov EMT-10 were lyophilized at varying temperatures.

The following samples were analyzed:
1. Neutral oil, unprocessed
2. Sepinov EMT-10, unprocessed
3. Hyaluronic acid, unprocessed
4. Sepinov EMT-10, lyophilized at 120 °C
5. Hyaluronic acid, lyophilized at 120 °C
6. Mixture (hyaluronic acid, neutral oil and Sepinov EMT-10), lyophilized at 40 °C
7. Mixture, lyophilized at 60 °C
8. Mixture, lyophilized at 80 °C
9. Mixture, lyophilized at 100 °C
10. Mixture, lyophilized at 120 °C

The samples were analyzed using size exclusion chromatography on an HPLC system, using 3 analytical columns. Samples were dissolved in PBS-Buffer with pH 7.4, non-soluble parts were removed by filtration.

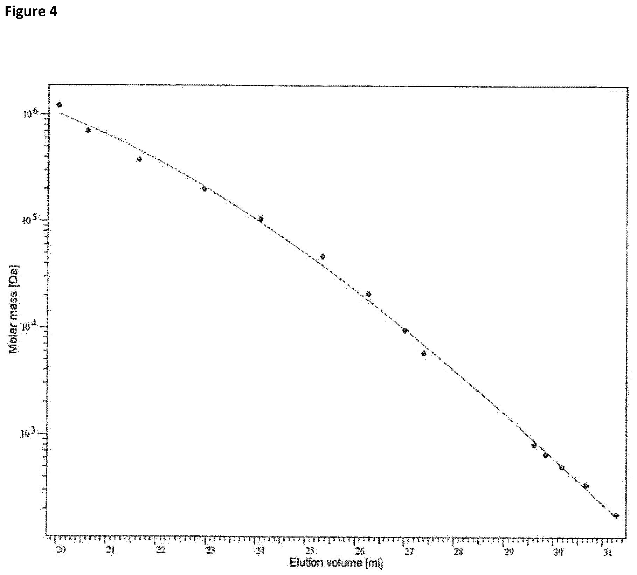

The columns were calibrated using dextran/pullulan standards. Molecular masses of the samples were determined based on the said calibration (for the calibration curve see Figure 4).

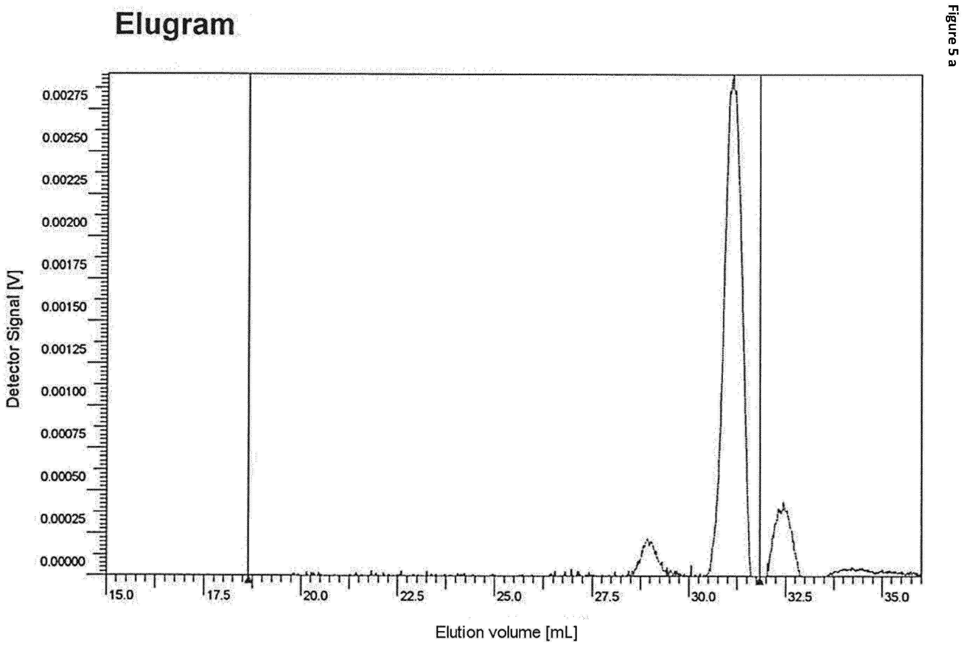

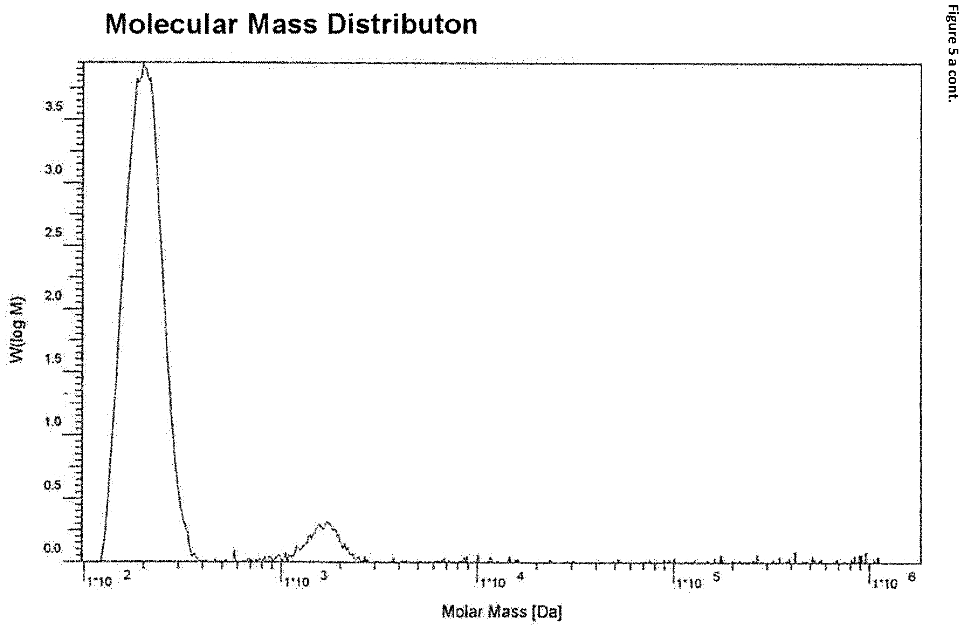

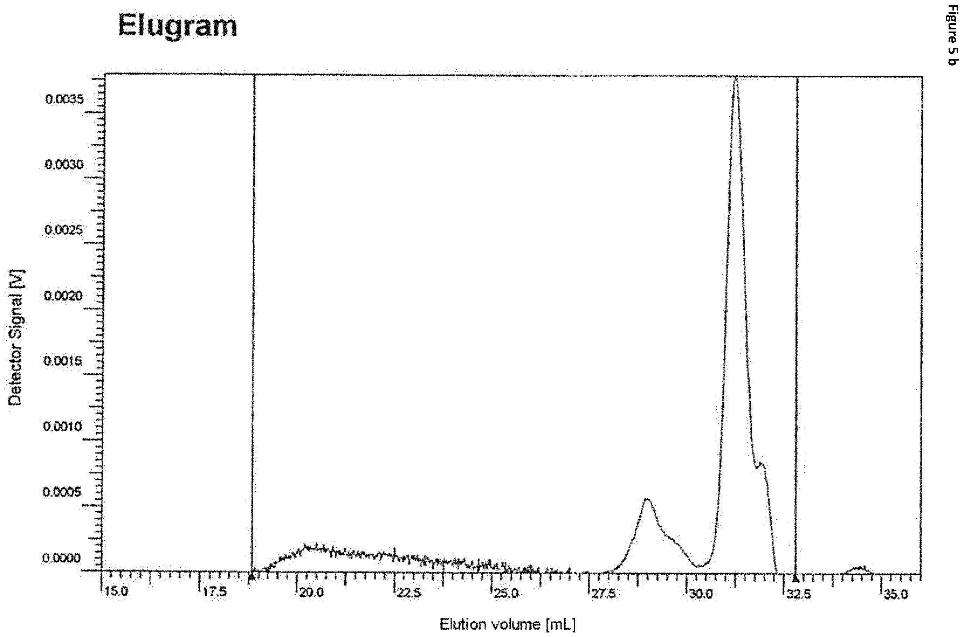

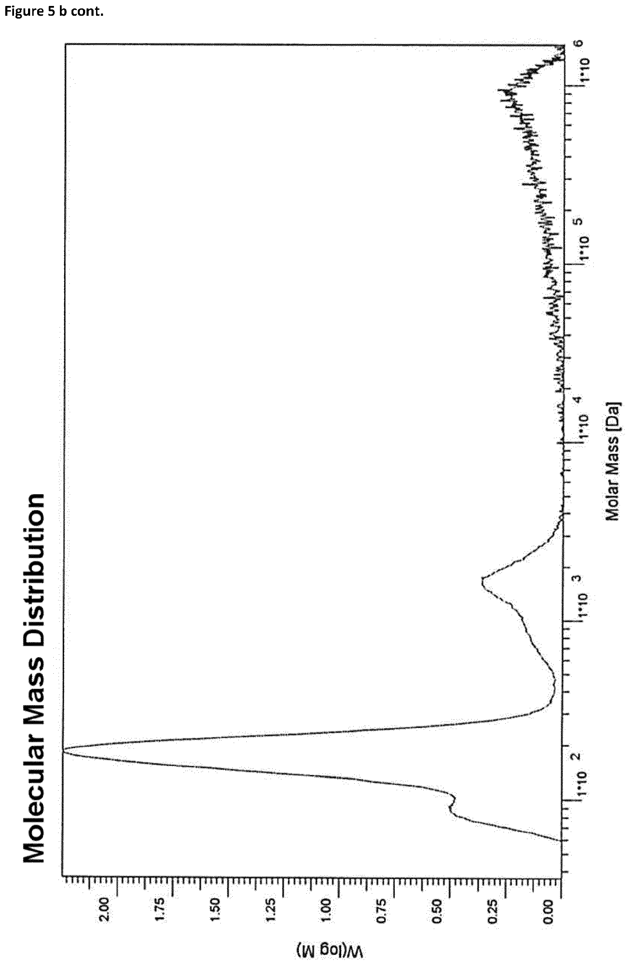

Only pure hyaluronic acid samples were completely soluble. The soluble components of the Sepinov EMT-10 or neutral oil, do not produce any problematic signals during analysis (see Figure 5 a and b).

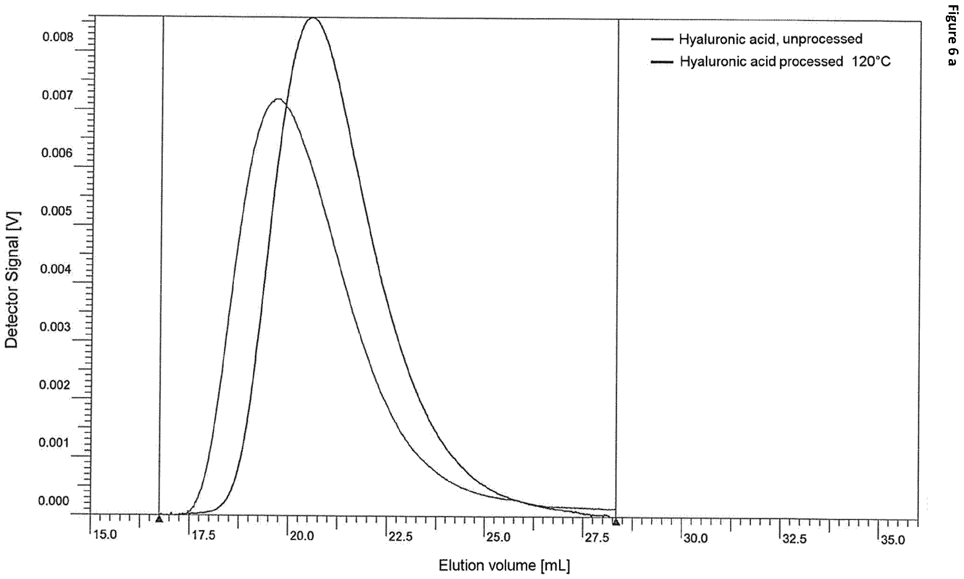

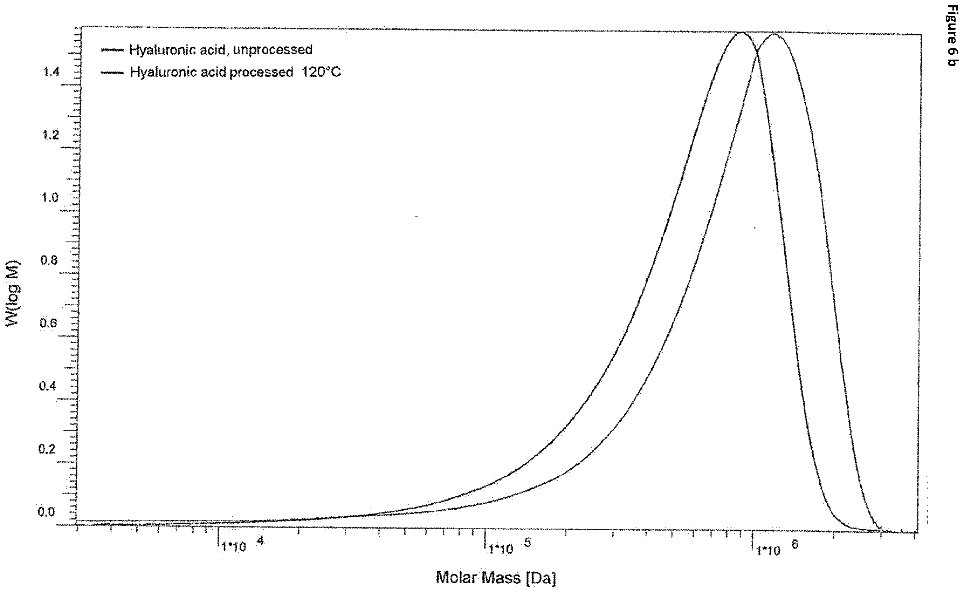

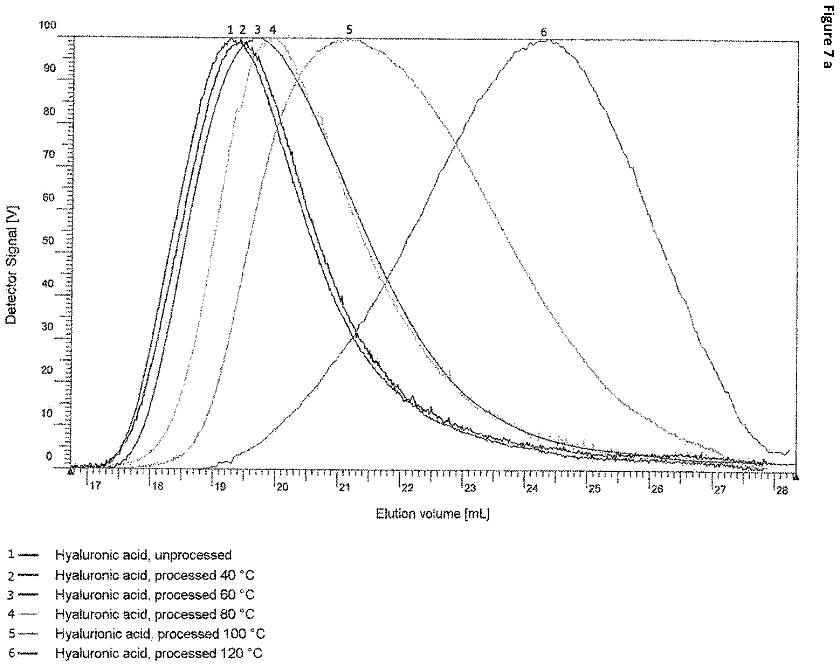

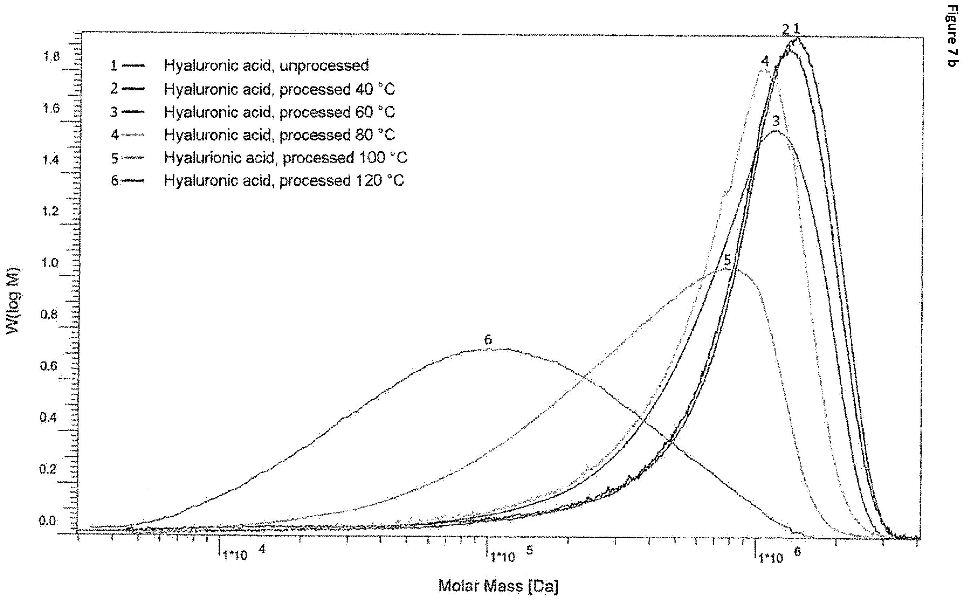

The results clearly show that the composition and the lyophilization temperature affect the average molecular weight of the hyaluronic acid. While an effect of lyophilization on pure hyaluronic acid at high temperatures occurs and results in a reduced average molecular weight (see Figures 6 a,b), the effect is stronger in the mixtures (see Figure 7 a,b).

Overall it is clearly visible that the choice of parameters during the lyophilization process is suitable to control the average molecular weight of hyaluronic acid after the lyophilization.

### Example 2: Influence of the pH-value on degradation

Hyaluronic acid with a molecular weight of 1.478 Mio Da (Contipro, Mw, according to gel permeation chromatography) was dissolved in 1 wt-% solution in distilled water at 80°C for five minutes. The pH was adjusted with hydrochloric acid in the range of 2.9 to 6.21.

7.5 ml HA solution was dispensed in 10 ml glass vials, samples were frozen at -20°C overnight and placed in a Christ Epsilon 2-10D LSC plus HT device and processed for approximately 20 hours according to the 10/120°C temperature profile shown in Figure 3.

Lyophilised samples were diluted in GPC buffer (pH 7.4) at a concentration of 0.3 wt-% and analyzed by means of gel permeation chromatography against Pullulan and Dextran molecular weight standards.

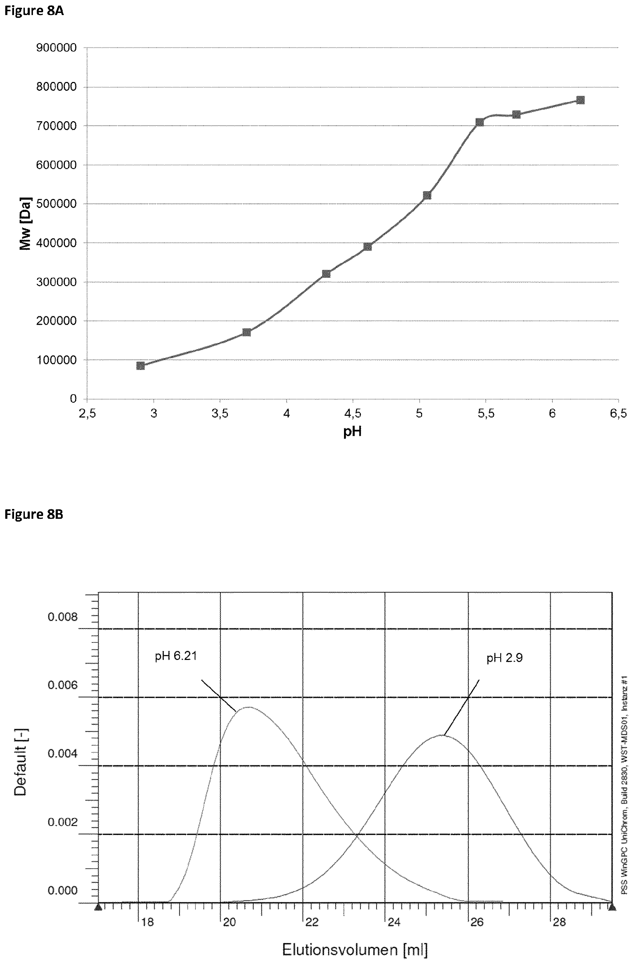

Independent on adjusted pH, all samples were cleaved showing a maximum of 766 kDa at pH 6.21 and a minimum 84.75 kDa at pH 2.9 (Figure 7). The higher the amount of free acid functionality in the polymer the higher the tendency of the polymer to be cleaved. A corresponding elugram of the high as well as the low molecular weight sample is shown in Figure 8.

### Example 3: Degradation of hyaluronic acids with different molecular weights

Four differents types of hyaluronic acid (Contipro/GfN 3010 (MW: 1478 kDa), Principium Cube3 (MW: 733 kDa), Principium Signal-10 (MW: 25 kDa) and Freda mini-HA (MW: 27 kDa)) were dissolved in 1 wt-% solution in distilled water at 80°C for five minutes. Solution were used as is or pH was adjusted to approximately 3.5.

7.5 ml HA solution was dispensed in 10 ml glass vials, samples were frozen at -20°C overnight and placed in a Christ Epsilon 2-10D LSC plus HT device and processed for approximately 20 hours according to the 10/120°C or alternatively the 120°C temperature profile shown in Figure 3.

Lyophilised samples were diluted in GPC buffer (pH 7.4) at a concentration of 0.3 wt-% and analysed by means of gel permeation chromatography against Pullulan and Dextran molecular weight standards.

High and medium molecular weight hyaluronic acid showed a moderate decay of molecular weight at original pH dissolved in distilled water, whereas molecular weight of substances decayed drastically at low pH, as shown in the following table.

| **Mw [kDa] non-processed** | **pH** | **Mw [kDa]* processed at 10/120°C** | **Mw [kDa]* processed at 120°C** |
|---|---|---|---|
| 1478 | 6.11 | 594 | 531 |
| 1478 | 3.50 | 97 | 68 |
| 733 | 6.63 | 331 | 297 |
| 733 | 3.57 | 97 | 78 |
| 25 | 3.36 | 23 | 21 |
| 27 | 6.53 | nd | 29 |
| 27 | 3.50 | nd | 28 |

### Example 4: Emulsions containing hyaluronic acid

5g of high molecular weight hyaluronic acid (GfN/Contipro 3010, 1.5 MDa) was dissolved in 465g of distilled water, heated to 80°C and stirred by means of a Somakon MP-LB (1l) mixing device at 1400 rpm and ambient pressure for 15 minutes.

7.5g Sepinov EMT-10 (INCI name: Hydroxyethyl acrylate (and) Sodium Acryloyl Dimethyl Taurate Copolymer) was added the pH was adjusted to 3.05 and mixture was stirred at 1400 rpm/200 µbar for further 15 minutes at 80°C.

25g of medium chain triglyciderides (MCTs) as model oil compound were added and homogenized at 2100 rpm/200µbar for 5 minutes.

7.5 ml of the resulting emulsion was dispensed in 10 ml glass vials, samples were frozen at -20°C overnight and placed in a Christ Epsilon 2-10D LSC plus HT device and processed for approximately 20 hours at maximum 40, 60, 80, 100 and 120°C.

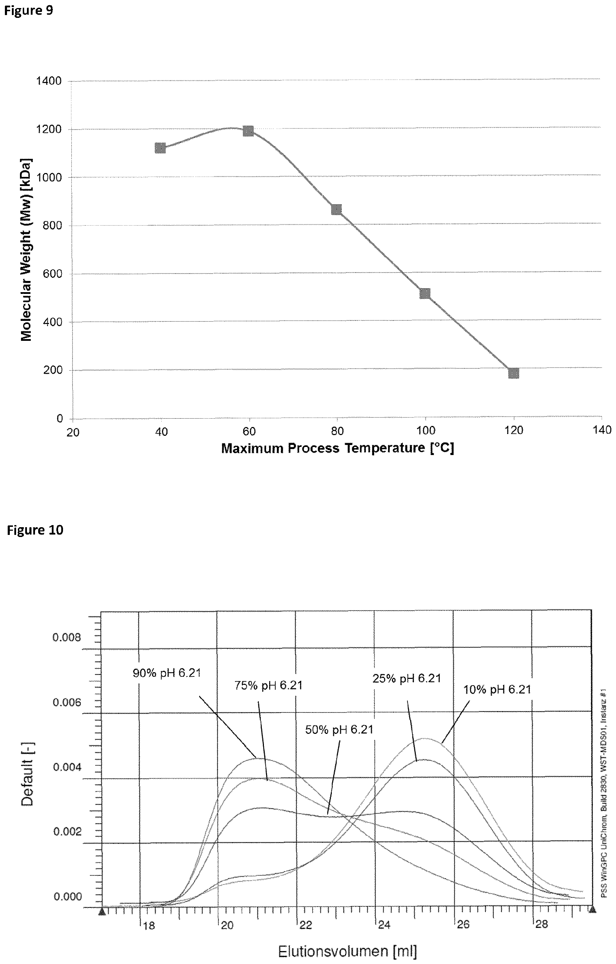

Lyophilised samples were diluted in GPC buffer (pH 7.4) at a concentration of 0.3 wt-% and analyzed by means of GPC. Figure 9 shows the temperature dependence of the molecular weight (Mw) of the hyaluronic acid decreasing with increasing maximum process temperature.

### Example 5: Lyophilization of different biopolymers

Polymers were dissolved in 1 wt-% solution in distilled water at 80°C for five minutes. The pH of the solutions was measured and the molecular weight distribution of the non-processed polymer solutions were determined by means of size exclusion chromatography against Pullulan and Dextran molecular weight standards diluting the samples to 0.3 wt-% in PBS buffer (pH 7.4).

7.5 ml polymer solution was dispensed in 10 ml glass vials, samples were frozen at -20°C overnight and placed in a Christ Epsilon 2-10D LSC plus HT device and processed for approximately 20 hours according to the 10/120°C temperature profile shown in Figure 3.

Lyophilised samples were diluted in GPC buffer (pH 7.4) at a concentration of 0.3 wt-% and analysed by means of GPC. The results are shown in the following tables.

### Sodium alginates:

| **Mw [kDa]* non-processed** | **pH** | **Mw [kDa] processed at 10/120°C** | **Mw [kDa] processed at 120°C** |
|---|---|---|---|
| 1074 | 6.88 | 336 | 269 |
| 1074 | 3.50 | 239 | nd |
| 1020 | 7.03 | 472 | 336 |
| 1020 | 3.50 | 287 | nd |
| 881 | 7.15 | 259 | 233 |
| 881 | 3.50 | 184 | nd |

### Polysaccharides:

| **Polymer** | **Monomers** | **pH** | **Mw [kDa]* non-processed** | **Mw [kDa]** processed at 10/120°C** | **Mw [kDa]** processed at 120°C** |
|---|---|---|---|---|---|
| Rhizobian Gum | tbd | 5.59 | 706 | 533 | 592 |
| | | 3.50 | 706 | 354 | nd |
| Sodium carboxy methyl cellulose | Funktionalized glucose | 6.66 | 682 | 689 | 712 |
| | | 3.5 | 682 | 309 | 308 |
| Pullulan | Glucose (Maltotriose) | 5.46 | 314 | 239 | 278 |
| | | 3.50 | 314 | 61 | nd |
| Biosaccharide Gum-1 | Fucose | 7.35 | 2037**** | 1735**** | 1598**** |
| | | 3.50 | 2037**** | 443 | nd |
| Glucomannane | Glucose, mannose | 5.84 | 1304 | 1119 | 980 |
| | | 3.50 | 1304 | 247 | nd |
| Beta-Glucan (and) Pectin | Galacturonic acid, rhamnose | 4.02 | 778 | 389 | 358 |
| Tamarindus indica Seed Polysaccharide | Glucose, xylose, galoctoxylose | 6.20 | 956 | 933 | 927 |
| | | 3.50 | 956 | 602 | nd |

### Example 6: pH and volume variation with stacked hyaluronic acid solutions

Hyaluronic acid with a molecular weight of 1.478 Mio Da (Contipro, Mw, according to gel permeation chromatography) was dissolved in 1 wt-% solution in distilled water at 80°C for five minutes. One fraction of the solution was used at normal pH, the second fraction was adjusted with hydrochloric acid to pH 2.9.

pH 6.21 HA solution was dispensed in differed volumes from 0.75 to 6.75 ml in 10 ml glass vials. Samples were frozen at -20°C and stacked with pH 2.9 HA solution at 6.75 to 0.75 ml and frozen again and stored at -20°C overnight. The corresponding volume ratios are shown in the following table:

| **Volume pH 6.21 solution [ml]** | **Volume pH 2.9 solution [ml]** | **Weight-% pH 6.21 solution** | **Weight-% pH 2.9 solution** | **Mw [kDa] of mixture** |
|---|---|---|---|---|
| 6.75 | 0.75 | 90 | 10 | 595 |
| 5.625 | 1.875 | 75 | 25 | 515 |
| 3.75 | 3.75 | 50 | 50 | 417 |
| 1.875 | 5.625 | 25 | 75 | 201 |
| 0.75 | 6.75 | 10 | 90 | 184 |

Samples were placed in a Christ 2-10D LSC plus HT device and processed for approximately 20 hours according to the 10/120°C temperature profile shown in Fig.3.

Lyophilised samples were diluted in GPC buffer (pH 7.4) at a concentration of 0.3 wt-% and analysed by means of gel permeation chromatography against Pullulan and Dextran molecular weight standards.

Dependant on volume ratio differently shaped molecular weight distrubutions can be shaped (see Fig. 10), indicating that dependent on the volume fractions and the adjusted pH in the volume fractions any shape of molecular weight distribution can be achived. Dependant on targeted biological function an optimum molecular weight distribution can be designed.

### Example 7: pH and volume variation with stacked pullulan solutions

Pullulan with a molecular weight of 371 kDa (Hayashibara, Mw, according to gel permeation chromatography) was dissolved in 1 wt-% solution in distilled water at 80°C for five minutes. One fraction of the solution was used at normal pH (4.9), the second fraction was adjusted with hydrochloric acid to pH 3.5.

pH 4.9 pullulan solution was dispensed in differed volumes from 0.75 to 6.75 ml in 10 ml glass vials. Samples were frozen at -20°C and stacked with pH 2.9 pullulan solution at 6.75 to 0.75 ml and frozen again and stored at -20°C overnight. The corresponding volume ratios are shown in the following table.

| **Volume pH 4.9 solution [ml]** | **Volume pH 3.5 solution [ml]** | **Weight-% pH 4.9 solution** | **Weight-% pH 3.5 solution** | **Mw [kDa] of mixture** |
|---|---|---|---|---|
| 6.75 | 0.75 | 90 | 10 | 176 |
| 5.625 | 1.875 | 75 | 25 | 133 |
| 3.75 | 3.75 | 50 | 50 | 94 |
| 1.875 | 5.625 | 25 | 75 | 62 |
| 0.75 | 6.75 | 10 | 90 | 36 |

Samples were placed in a Christ 2-10D LSC plus HT device and processed for approximately 20 hours according to the 10/120°C temperature profile shown in Fig.3.

Lyophilised samples were diluted in GPC buffer (pH 7.4) at a concentration of 0.3 wt-% and analysed by means of gel permeation chromatography against Pullulan and Dextran molecular weight standards.

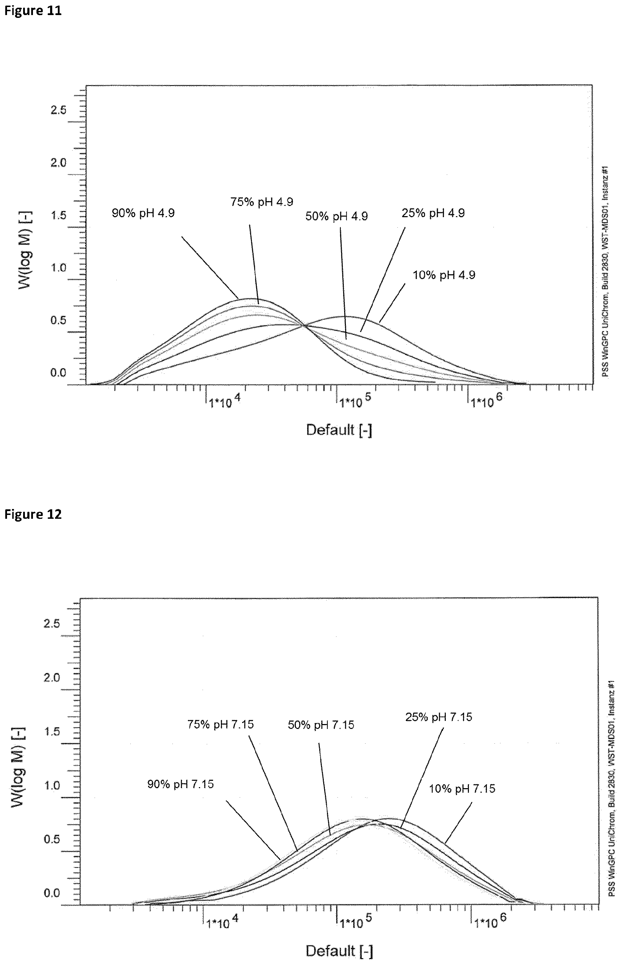

Dependant on volume ratio differently shaped molecular weight distrubutions can be achieved (see Fig. 11). Molecular weight Mw is mainly influenced by the amount of the low pH solution.

### Example 8: pH and volume variation with stacked sodium alginate solutions

Sodium alginate with a molecular weight of 881 kDa (Cargill, Mw, according to gel permeation chromatography) was dissolved in 1 wt-% solution in distilled water at 80°C for five minutes. One fraction of the solution was used at normal pH (7.15), the second fraction was adjusted with hydrochloric acid to pH 3.5.

pH 7.15 sodium alginate solution was dispensed in differed volumes from 0.75 to 6.75 ml in 10 ml glass vials. Samples were frozen at -20°C and stacked with pH 3.5 sodium alginate solution at 6.75 to 0.75 ml and frozen again and stored at -20°C overnight. The corresponding volume ratios are shown in the following table.

| **Volume pH 7.15 solution [ml]** | **Volume pH 3.5 solution [ml]** | **Weight-% pH 7.15 solution** | **Weight-% pH 3.5 solution** | **Mw [kDa] of mixture** |
|---|---|---|---|---|
| 6.75 | 0.75 | 90 | 10 | 357 |
| 5.625 | 1.875 | 75 | 25 | 315 |
| 3.75 | 3.75 | 50 | 50 | 278 |
| 1.875 | 5.625 | 25 | 75 | 253 |
| 0.75 | 6.75 | 10 | 90 | 245 |

Samples were placed in a Christ 2-10D LSC plus HT device and processed for approximately 20 hours according to the 10/120°C temperature profile shown in Fig. 3.

Lyophilised samples were diluted in GPC buffer (pH 7.4) at a concentration of 0.3 wt-% and analysed by means of gel permeation chromatography against Pullulan and Dextran molecular weight standards. Dependent on volume ratio differently shaped molecular weight distributions can be shaped (see Fig. 12). Shift in molelecur weight is mainly affected by the lyophilisation conditions and less by the amount of the low pH solution.

## Claims

1. Method for the production of a biopolymer composition comprising at least one biopolymer, wherein the at least one biopolymer has a defined average molecular weight, the method comprising
(i) providing a first frozen composition comprising a biopolymer with a defined pH-value;
(ii) providing a second frozen composition comprising a biopolymer with a defined pH-value
(iii) combining the compositions in a reaction vessel without substantially mixing these with each other;
(iv) optionally freezing the compositions;
(v) lyophylizing the compositions comprising the biopolymers,
(vi) optionally purifying and/or isolating the biopolymers;
wherein the maximum temperature during the lyophilization process is selected to facilitate a controlled and defined degradation of said biopolymer and wherein the pH-values of the first and second composition differ by at least 0.1 and/or wherein the first and second composition comprise different biopolymers.

2. The method according to claim 1, wherein the first and second composition comprise the same biopolymer.

3. The method according to claim 1, wherein the first and second composition comprise different biopolymers.

4. The method according to any of the claims 1 to 3, wherein the first and/or second composition comprising a biopolymer is a frozen aqueous solution or an emulsion.

5. The method according to any of the claims 1 to 4, wherein the pH value of the first and/or second composition is selected from a range between 1.5 and 8.5, preferably between 2.5 and 6.

6. The method according to any of the preceding claims, wherein the temperature during the sublimation process is selected from a range between -40 °C and 150 °C.

7. The method according to any of the preceding claims, wherein the pressure during the sublimation process is selected from a range between 50 µbar and 800 µbar.

8. The method according to any of the preceding claims, wherein the biopolymer of the first and/or second composition is selected from the group comprising hyaluronic acid, collagen, glucosamino glycans, polysaccharides and fucoidanes, preferably the biopolymer is hyaluronic acid.

9. The method according to claims 1 to 8, wherein the first and/or second composition is an emulsion comprising:
(i) a biopolymer,
(ii) water,
(iii) optionally a pharmaceutically, dermatologically and/or cosmetically acceptable compound or oil,
(iv) optionally an emulsifying agent and
(v) optionally emollients.

10. The method according to any of claims 1 to 9, wherein the first and/or second composition additionally comprises further dermatological, pharmaceutical or cosmetic additions.

11. The method according to any of the claims 1 to 10, wherein the first and/or second composition comprises additional components, so that the resulting lyophilized product is easily dissolvable or easily forms an emulsion.

12. The method according to any of the claims 1 to 11, wherein the first and second composition comprise the same contents and differ only in pH-value and/or the biopolymer.

13. The method according to any of the claims 1 to 12, wherein the first and second composition are combined in an appropriate container, which is suitable for the lyophilization process and which can serve as primary packaging.

14. The method according to any of claims 1 to 13, wherein the biopolymer of the first and/or second composition is selected from the group comprising hyaluronic acid, collagen, glucosamino glycans, polysaccharides and fucoidanes, preferably the biopolymer is hyaluronic acid.

15. Use of a method according to any of the claims 1 to 14 for the production of a pharmaceutically, dermatologically or cosmetically acceptable biopolymer composition.

## Patentansprüche

1. Verfahren zur Herstellung einer Biopolymerzusammensetzung, die mindestens ein Biopolymer umfasst, wobei das mindestens eine Biopolymer ein definiertes durchschnittliches Molekulargewicht aufweist, wobei das Verfahren umfasst
(i) Bereitstellen einer ersten gefrorenen Zusammensetzung, die ein Biopolymer umfasst, mit einem definierten pH-Wert;
(ii) Bereitstellen einer zweiten gefrorenen Zusammensetzung, die ein Biopolymer umfasst, mit einem definierten pH-Wert;
(iii) Kombinieren der Zusammensetzungen in einem Reaktionsgefäß, ohne diese wesentlich miteinander zu vermischen;
(iv) optionales Einfrieren der Zusammensetzungen;
(v) Lyophilisieren der Zusammensetzungen, die die Biopolymere enthalten,
(vi) optionales Reinigen und/oder Isolieren der Biopolymere;
wobei die maximale Temperatur während des Gefriertrocknungsprozesses so gewählt wird, dass ein kontrollierter und definierter Abbau des Biopolymers ermöglicht wird, und wobei die pH-Werte der ersten und zweiten Zusammensetzung sich um mindestens 0,1 unterscheiden und/oder wobei die erste und zweite Zusammensetzung unterschiedliche Biopolymere umfassen.

2. Verfahren nach Anspruch 1, wobei die erste und die zweite Zusammensetzung das gleiche Biopolymer umfassen.

3. Verfahren nach Anspruch 1, wobei die erste und die zweite Zusammensetzung unterschiedliche Biopolymere umfassen.

4. Verfahren nach einem der Ansprüche 1 bis 3, wobei die erste und/oder zweite Zusammensetzung, die ein Biopolymer umfasst, eine gefrorene wässrige Lösung oder eine Emulsion ist.

5. Verfahren nach einem der Ansprüche 1 bis 4, wobei der pH-Wert der ersten und/oder zweiten Zusammensetzung aus einem Bereich zwischen 1,5 und 8,5, vorzugsweise zwischen 2,5 und 6, ausgewählt wird.

6. Verfahren nach einem der vorhergehenden Ansprüche, wobei die Temperatur während des Sublimationsprozesses aus einem Bereich zwischen -40 °C und 150 °C ausgewählt wird.

7. Verfahren nach einem der vorhergehenden Ansprüche, wobei der Druck während des Sublimationsprozesses aus einem Bereich zwischen 50 µbar und 800 µbar ausgewählt wird.

8. Verfahren nach einem der vorhergehenden Ansprüche, wobei das Biopolymer der ersten und/oder zweiten Zusammensetzung aus der Gruppe umfassend Hyaluronsäure, Kollagen, Glucosaminoglykane, Polysaccharide und Fucoidane ausgewählt wird, wobei das Biopolymer vorzugsweise Hyaluronsäure ist.

9. Verfahren nach einem der Ansprüche 1 bis 8, wobei die erste und/oder zweite Zusammensetzung eine Emulsion ist, umfassend:
(i) ein Biopolymer,
(ii) Wasser,
(iii) gegebenenfalls eine pharmazeutisch, dermatologisch und/oder kosmetisch akzeptable Verbindung oder ein ÖI,
(iv) gegebenenfalls ein Emulgiermittel und
(v) gegebenenfalls Emollienzien.

10. Verfahren nach einem der Ansprüche 1 bis 9, wobei die erste und/oder zweite Zusammensetzung zusätzlich weitere dermatologische, pharmazeutische oder kosmetische Zusätze enthält.

11. Verfahren nach einem der Ansprüche 1 bis 10, wobei die erste und/oder zweite Zusammensetzung zusätzliche Komponenten umfasst, so dass das resultierende lyophilisierte Produkt leicht auflösbar ist oder leicht eine Emulsion bildet.

12. Verfahren nach einem der Ansprüche 1 bis 11, wobei die erste und die zweite Zusammensetzung die gleichen Inhaltsstoffe umfassen und sich nur im pH-Wert und/oder dem Biopolymer unterscheiden.

13. Verfahren nach einem der Ansprüche 1 bis 12, wobei die erste und die zweite Zusammensetzung in einem geeigneten Behälter, der für den Gefriertrocknungsprozess geeignet ist und der als Primärverpackung dienen kann, zusammengeführt werden.

14. Verfahren nach einem der Ansprüche 1 bis 13, wobei das Biopolymer der ersten und/oder zweiten Zusammensetzung aus der Gruppe umfassend Hyaluronsäure, Kollagen, Glucosaminoglykane, Polysaccharide und Fucoidane ausgewählt wird, wobei das Biopolymer vorzugsweise Hyaluronsäure ist.

15. Verwendung eines Verfahrens nach einem der Ansprüche 1 bis 14 zur Herstellung einer pharmazeutisch, dermatologisch oder kosmetisch akzeptablen Biopolymerzusammensetzung.

## Revendications

1. Procédé pour la production d'une composition de biopolymère comprenant au moins un biopolymère, dans lequel l'au moins un biopolymère a une masse moléculaire moyenne définie, le procédé comprenant
(i) l'obtention d'une première composition congelée comprenant un biopolymère ayant une valeur de pH définie ;
(ii) l'obtention d'une seconde composition congelée comprenant un biopolymère ayant une valeur de pH définie
(iii) la combinaison des compositions dans une cuve de réaction pratiquement sans mélange de celles-ci l'une avec l'autre ;
(iv) éventuellement la congélation des compositions ;
(v) la lyophilisation des compositions comprenant les biopolymères,
(vi) éventuellement la purification et/ou l'isolement des biopolymères ;
dans lequel la température maximale pendant le processus de lyophilisation est choisie pour faciliter une dégradation contrôlée et définie dudit biopolymère et dans lequel les valeurs de pH des première et seconde compositions diffèrent d'au moins 0,1 et/ou dans lequel les première et seconde compositions comprennent des biopolymères différents.

2. Procédé selon la revendication 1, dans lequel les première et seconde compositions comprennent le même biopolymère.

3. Procédé selon la revendication 1, dans lequel les première et seconde compositions comprennent des biopolymères différents.

4. Procédé selon l'une quelconque des revendications 1 à 3, dans lequel la première composition et/ou la seconde composition comprenant un biopolymère sont des solutions aqueuses congelées ou des émulsions.

5. Procédé selon l'une quelconque des revendications 1 à 4, dans lequel la valeur de pH de la première composition et/ou de la seconde composition est choisie dans une plage comprise entre 1,5 et 8,5, de préférence entre 2,5 et 6.

6. Procédé selon l'une quelconque des revendications précédentes, dans lequel la température pendant le processus de sublimation est choisie dans une plage comprise entre -40 °C et 150 °C.

7. Procédé selon l'une quelconque des revendications précédentes, dans lequel la pression pendant le processus de sublimation est choisie dans une plage comprise entre 50 µbar et 800 µbar.

8. Procédé selon l'une quelconque des revendications précédentes, dans lequel le biopolymère de la première composition et/ou de la seconde composition est choisi dans le groupe comprenant l'acide hyaluronique, le collagène, les glucosaminoglycanes, les polysaccharides et les fucoïdanes, de préférence le biopolymère est l'acide hyaluronique.

9. Procédé selon les revendications 1 à 8, dans lequel la première composition et/ou la seconde composition sont des émulsions comprenant :
(i) un biopolymère,
(ii) de l'eau,
(iii) éventuellement un composé ou une huile pharmaceutiquement, dermatologiquement et/ou cosmétiquement acceptable,
(iv) éventuellement un agent émulsifiant et
(v) éventuellement des émollients.

10. Procédé selon l'une quelconque des revendications 1 à 9, dans lequel la première composition et/ou la seconde composition comprennent de plus d'autres adjuvants dermatologiques, pharmaceutiques ou cosmétiques.

11. Procédé selon l'une quelconque des revendications 1 à 10, dans lequel la première composition et/ou la seconde composition comprennent des composants supplémentaires, pour que le produit lyophilisé résultant soit facilement soluble ou forme facilement une émulsion.

12. Procédé selon l'une quelconque des revendications 1 à 11, dans lequel les première et seconde compositions comprennent les mêmes teneurs et diffèrent uniquement en ce qui concerne la valeur de pH et/ou le biopolymère.

13. Procédé selon l'une quelconque des revendications 1 à 12, dans lequel les première et seconde compositions sont combinées dans un récipient approprié, qui convient pour le processus de lyophilisation et qui peut servir d'emballage primaire.

14. Procédé selon l'une quelconque des revendications 1 à 13, dans lequel le biopolymère de la première composition et/ou de la seconde composition est choisi dans le groupe comprenant l'acide hyaluronique, le collagène, les glucosaminoglycanes, les polysaccharides et les fucoïdanes, de préférence le biopolymère est l'acide hyaluronique.

15. Utilisation d'un procédé selon l'une quelconque des revendications 1 à 14 pour la production d'une composition de biopolymère pharmaceutiquement, dermatologiquement ou cosmétiquement acceptable.
